# EUROPEAN PATENT SPECIFICATION

(11) **EP 0 497 976 B1**
(45) Date of publication and mention of the grant of the patent: **11.09.1996**
(21) Application number: 90912467.9
(22) Date of filing: 27.08.1990
(51) Int. Cl.: C07D 493/04

(54) **METHOD OF PRODUCING ACETALS**
VERFAHREN ZUR HERSTELLUNG VON ACETALEN
PROCEDE DE PRODUCTION DE COMPOSES D'ACETAL

(43) Date of publication of application: 12.08.1992
(73) Proprietor: NEW JAPAN CHEMICAL CO.,LTD., Kyoto-shi Kyoto 612 (JP)
(72) Inventor: KOBAYASHI, Toshiaki, 11-16, Suzaku 5-chome, Nara 631 (JP); KITAGAWA, Sachio, 3-A-15-102, Takezono Otokoyama, Kyoto 614 (JP); SAKURAI, Shozo, 29-13, Minami-maioka 1-chome, Kanagawa 244 (JP)
(74) Representative: VOSSIUS & PARTNER
(86) International application number: JP9001086
(87) International publication number: WO9203439

(56) References cited:
- JP-A- 1 149 789
- JP-A- 5 035 163
- JP-A-56 108 791
- JP-A-56 161 391
- JP-A-57 185 288
- JP-A-58 180 488
- JP-B- 4 843 748

## Description

The present invention relates to a process for producing acetal compounds such as dibenzylidene-sorbitols and nucleus-substituted products thereof, and more particularly to an industrially advantageous process for producing acetals with a high efficiency by a facilitated procedure.

Acetal compounds such as dibenzylidenesorbitols, dibenzylidenexylitols and nucleus-substituted products thereof possess peculiar properties as additives and have found wide use as transparency improving agents and nucleating agents for polypropylene resins, polyethylene resins, polyethylene terephthlate resins and the like; heat-resistant flowability imparting agents for polymers; flowability improving agents and thixotropic agents for paints, ink and adhesives; and solidifying agents for adhesives, cosmetics and pharmaceuticals.

These acetal compounds are prepared usually by the condensation of an aromatic aldehyde with a polyhydric alcohol such as sorbitol or xylitol (the aromatic aldehyde compound and the polyhydric alcohol will hereinafter be referred to as "substrates").

Our research has revealed that the acetal compound prepared by the conventional process includes not only isomers which are effective as the above-mentioned additives, i.e., 1,3;2,4-didioxane-type isomers represented by the general formula 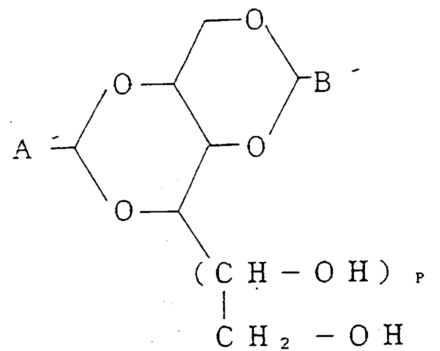 wherein A' and B' are the same or different and each represents a substituted or unsubstituted aromatic ring group, and p is 0 or 1, but also monodioxane-type isomers represented by the general formula 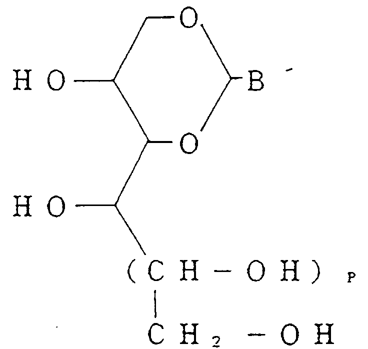 wherein B' and p are as defined above, and dioxolan-type isomers represented by the general formulae 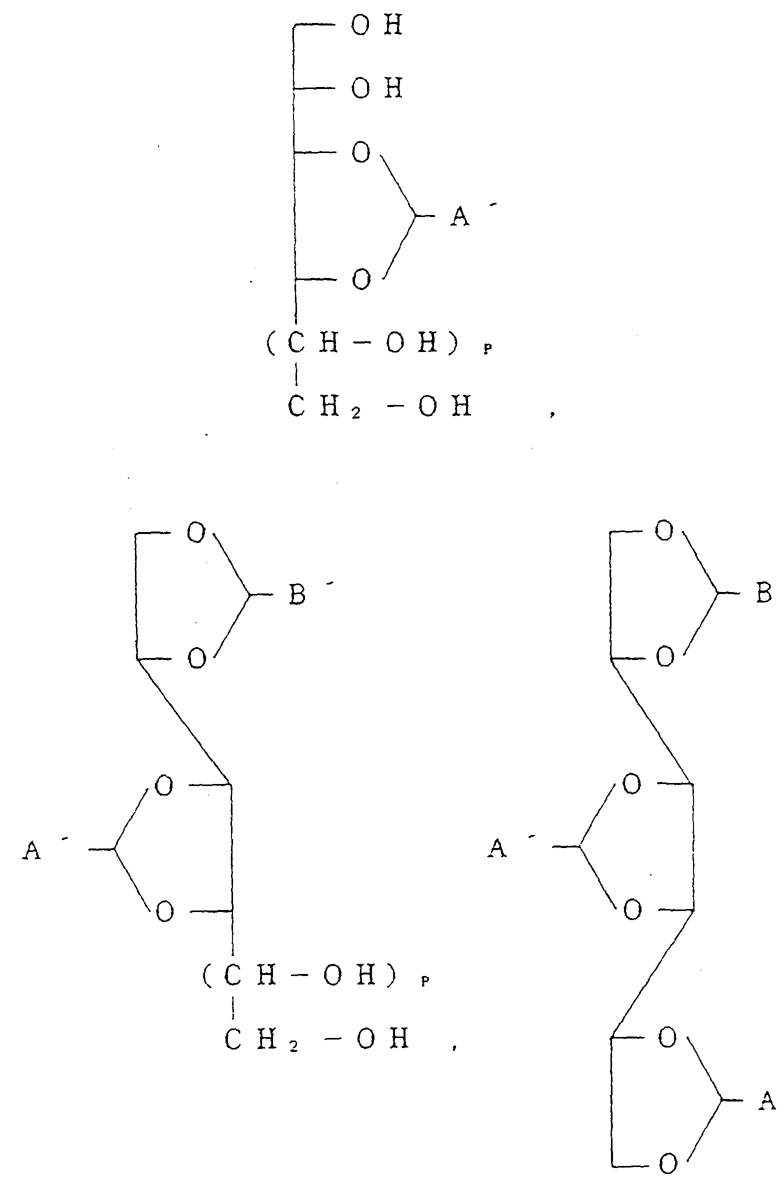 wherein A', B' and p are as defined above.

When the acetal compound is used as a transparency improving agent or nucleating agent for resins, the dioxolan-type isomers, which are thermally unstable, often decompose, consequently releasing a noxious odor or coloring the resin. To reduce the content of the isomer has therefore been a very important subject of research.

We have made detailed evaluation of conventional production processes directing attention to the preparation of the desired product with a high purity and good reproducibility as well as to the reduction of the content of dioxolan-type isomers, achievement of a higher reaction yield and simplification of the process, and consequently found that the conventional processes have the following problems.

### (1) Process of Examined Japanese Patent Publication SHO 48-43748

In this process, sorbitol is reacted with benzaldehyde in the presence of cyclohexane and one or at least two of C₆ to C₁₆ saturated hydrocarbons using a water-soluble organic solvent when so required to prepare dibenzylidenesorbitol or a mixture thereof with mono-benzylidenesorbitol. This process is considerably useful when the reaction system is up to about 15 wt. % in the slurry concentration of the product but inevitably permits formation of about 6 to about 20 wt. % of dioxolan-type isomers as by-products. Accordingly, the process requires purification with use of methanol or methyl ethyl ketone to remove the isomers. Further, the use of a lower alcohol as the water-soluble organic solvent still fails to achieve a fully satisfactory conversion, similarly permitting inevitable formation of dioxolan-type isomers as by-products.

### (2) Process of Examined Japanese Patent Publication SHO 58-22157

With this process, the substrates of high concentration are subjected to condensation with forced agitation in the presence of an acid catalyst, cyclohexane or like hydrophobic organic solvent and lower alcohol or like water-soluble polar organic solvent. Although superior in productivity, the process involves the likelihood that locally the acid catalyst will not be neutralized uniformly when the resulting reaction mixture in the form of a gel is purified for the isolation of the desired product because of the high concentration of the substrates. As a result, the product is unstable in quality, for example, owing to hydrolysis which occurs during a drying step to lower the purity. Moreover, a low yield will result not infrequently. Therefore, a production operation is unstable.

### (3) Process of European Patent Laid-Open Application No. 0319917 (EP 0319917A2)

With this process, a slurry (pre-reaction mixture) of the substrates and a lower alcohol is reacted while being continuously or intermittently charged into a reactor. The drawback of the process of the foreging publication SHO 58-22157 is overcome by this process to a considerable extent. However, the charging rate of the material needs to be controlled to make the mean resident time constant (about 2 hours) if high selectivity and high conversion are to be maintained at all times. For this reason, the charging rate in the initial to middle stages of the reaction is extremely low, necessitating a long period of time for the reaction and resulting in a low production efficiency. The charging rate, if great, entails undesirable results such as a reduced conversion, formation of a gel and production of dioxolan-type isomers. The later stage of reaction requires a very high charging rate of the pre-reaction mixture to inevitably entail a lower conversion.

Thus, the prior art still remains to be fully improved in production efficiency, necessitates a cumbersome technique for controlling the reaction and has other problems.

We conducted intensive research to produce the desired acetal compound with a remarkably improved efficiency and found the following.
(1) If the amount of lower alcohol present in the reaction system is too small, a reduced conversion and lower selectivity result.
(2) An excess of lower alcohol, if present in the reaction system, necessitates a prolonged length of time for the completion of reaction, making it impossible to effectively separate an upper layer from a lower layer in a decanter, thereby failing to efficiently draw off water from the reaction system and to achieve a fully satisfactory conversion.

We have further carried out continued research and found that the desired product can be prepared with a remarkably increased reaction efficiency and also with improved selectivity by the simplified procedure of adding a divided portion of a lower alcohol to the reaction system, subsequently drawing off the added lower alcohol and the resulting water from the reaction mixture by vapor-liquid equilibration or in the form of an azeotropic mixture, further adding a suitable amount of the lower alcohol to the system, drawing off the lower alcohol and the resulting water from the reaction system and thereafter repeating these steps a required number of times not less frequent than three times instead of permitting the progress of the reaction merely in the presence of the lower alcohol. The invention has been accomplished based on this finding.

More specificaly, the present invention provides a process for producing an acetal compound represented by the general formula 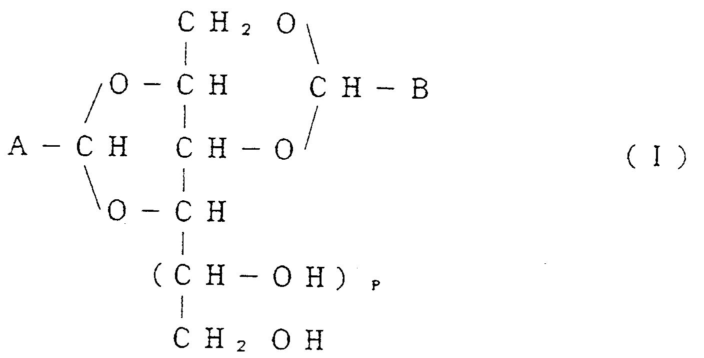 wherein A and B are the same or different and each represents phenyl, naphthyl or tetrahydronaphthyl, the phenyl, naphthyl or tetrahydronaphthyl having or not having 1 to 5 substitutents selected from among C₁-C₄ alkyl group, C₁-C₄ alkoxy group, halogen atom, carboxyl group, (C₁-C₂₀ alkyloxy)carbonyl group, (C₁-C₂₀ alkyloxy)ethyloxycarbonyl group, (C₁-C₁₂ alkyl)phenyl group, halogenated phenyl group, (C₁-C₁₂ alkoxy)phenyl group, (C₁-C₁₂ alkyloxy)ethyloxyethyloxycarbonyl group and (C₁-C₁₂ alkyloxy)ethyloxyethyloxyethyloxycarbonyl group, and p is 0 or 1 by subjecting (A) at least one of aldehyde compounds represented by the general formula

A-CHO (II)

and the general formula

B-CHO (III)

wherein A and B are as defined above, and (B) a polyhydric alcohol selected from among a pentahydric alcohol and a hexahydric alcohol to condensation in the presence of (C) a lower alcohol selected from the group consisting of a saturated aliphatic alcohol having 1 to 4 carbon atoms, allyl alcohol or furfuryl alcohol, (D) a hydrophobic organic solvent not forming a gel in any way or not forming a tough gel with the acetal compound of the general formula (I) prepared, serviceable as a dispersion medium and having a boiling point of about 40 to about 200°C and (E) an acid catalyst, the process being characterized in that the procedure of charging the lower alcohol into a reactor and thereafter withdrawing more than one-half of the charge of lower alcohol from the reaction system along with water is repeated at least three times during the condensation reaction.

The acetal compounds of the general formula (I) thus prepared include those of the symmetric type wherein the two aromatic rings thereof are identical in the kind and number of substituents thereon, and those of the asymmetric type wherein the rings have different substituents.

The present invention overcomes the problems of the prior art, e.g., the problem of being unsatisfactory in production efficiency and necessitating a cumbersome technique for controlling the reaction. Especially, the invention obviates leveling-off of the reaction efficiency and makes it possible for the reaction to proceed quantitatively. Moreover, almost 100% selectivity of the desired product can be achieved presumably because almost all the dioxolan-type isomers formed as by-products during the reaction are converted one after another into dioxane-type isomers. The invention further has the advantage that a semitransparent gel-like aggregate (gel of aldehyde compound and various isomers) once formed during production is also dispersed favorably during continued reaction for the progress of reaction.

The aldehyde compounds (A) of the general formulae (II) and (III) for use in the present process as one of the starting materials are benzaldehyde, naphthaldehyde and 1,2,3,4-tetrahydronaphthaldehyde which may have one to five, preferably one, two or three substituents selected from among C₁-C₄ alkyl group, C₁-C₄ alkoxy group, halogen atom, carboxyl group, (C₁-C₂₀ alkyloxy)carbonyl group, (C₁-C₂₀ alkyloxy)ethyloxycarbonyl group, (C₁-C₁₂ alkyl)phenyl group, halogenated phenyl group, (C₁-C₁₂ alkoxy)phenyl group, (C₁-C₁₂ alkyloxy)ethyloxyethyloxycarbonyl group and (C₁-C₁₂ alkyloxy)ethyloxyethyloxyethyloxycarbonyl group. These compounds may be used singly or in the form of a desired mixture.

Preferable among the aldehydes (A) of the general formulae (II) and (III) are benzaldehyde which may have a substituent or substituents and unsubstituted tetrahydronaphthylaldehyde, examples of such substituents being p-methyl, m-methyl, o-methyl, p-ethyl, p-isopropyl, p-butyl, p-isobutyl, p-phenyl, p-(C₁-C₁₂ alkyl)phenyl, p-halogenated phenyl, p-(C₁-C₁₂ alkoxy)-phenyl, 2,4-dimethyl, 2,4,5-trimethyl, p-chloro, p-methoxy, p-fluoro, p-methoxycarbonyl, p-hexyloxycarbonyl, p-butyloxyethyloxycarbonyl and p-methyloxyethyloxyethyloxycarbonyl.

The polyhydric alcohol (B) selected from among a pentahydric alcohol and a hexahydric alcohol for use as the other starting material is represented by the general formula 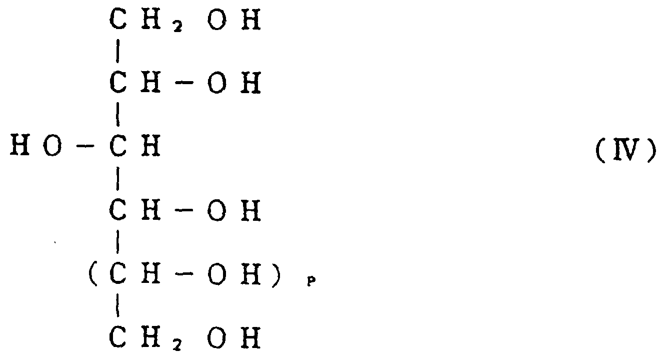 wherein p is 0 or 1. Typically, preferable to use are sorbitol and xylitol, which may be used in an optional ratio as mixture.

The hydrophobic organic solvent (D) to be used in the invention can be any of those which will not gel in any way or form a tough gel by the action of the acetal compound of the formula (I) obtained and which serve as a dispersion medium. Preferable are generally those having a boiling point of about 40 to about 200°C. Especially in view of the ease of distilling off water and the alcohol from the reaction system in the form of an azeotropic mixture composed of the three components of water, alcohol and hydrophobic organic solvent, it is desirable to use benzene which may be substituted with one to three C₁-C₄ alkyl groups, such as benzene, toluene and xylene; cyclohexane which may be substituted with one to three C₁-C₄ alkyl groups, such as cyclohexane, methylcyclohexane and ethylcyclohexane; straight- or branched-chain saturated hydrocarbons having 6 to 16, preferably 6 to 12, carbon atoms such as hexane, heptane, octane, nonane, decane, undecane and dodecane.

Examples of lower alcohols (C) are saturated aliphatic alcohols having 1 to 4 carbon atoms, such as methanol, ethanol, n-propanol, isopropanol and butanol, allyl alcohol or furfuryl alcohol .

An acid catalyst (E) is used in the present invention. The acid catalyst to be used is any of usual Brønsted acids and Lewis acids. Examples of such acids are sulfuric acid, phosphoric acid, hydrochloric acid, p-toluenesulfonic acid, m-toluenesulfonic acid, benzenesulfonic acids substituted with an alkyl group having 2 to 18 carbon atoms, naphthalenesulfonic acid, G acid, R acid, zinc chloride, etc.

The production process of the invention is practiced generally by charging into a reactor a mixture of:
(A) at least one of aldehyde compounds represented by the general formulae (II) and (III),
(B) a polyhydric alcohol represented by the general formula (IV),
(C) a lower alcohol,
(D) a hydrophobic organic solvent, and
(E) an acid catalyst,
and performing the procedure comprising the following steps (a) and (b) at least three times during the reaction until a desired selectivity of the compound of the general formula (I) is achieved, the step (a) being charging the lower alcohol into the reactor, the step (b) being withdrawing from the reaction system more than one-half of the amount of the lower alcohol and the water resulting from a condensation reaction, by vapor-liquid equilibration or as an azeotropic mixture.

The reactor to be used is not limited specifically but can be any of those generally used. For example, a reactor equipped with a condenser having a decanter, thermometer, gas inlet, stirrer, etc. is usable.

The charge mole ratio of aldehyde compound (A) to polyhydric alcohol (B) is not limited specifically but can be suitably determined from a wide range. The ratio (A):(B) is in the range of about 1-4:1. However, to ensure improved selectivity of the desired acetal compound of the formula (I), the ratio (A):(B) is preferably about 1.5-3:1, more preferably about 1.8-2.2:1.

The whole amounts of the substrates ((A) and (B)) may be charged into the reactor from the beginning. Alternatively, a mixture of aldehyde compound (A) and polyhydric alcohol (B) in the above ratio is first mixed with the lower alcohol (C) to prepare a uniform solution or dispersion (hereinafter referred to as a "pre-reaction mixture"), which may be charged continuously or intermittently. Presumably, the pre-reaction mixture contains a mixture of acetals formed by the reaction of the three compounds, i.e., the aldehyde compound (A), polyhydric alcohol (B) and lower alcohol (C). The amount of lower alcohol (C) to be used for preparing the pre-reaction mixture is suitably determined from the range of about 0.1 to about 5 parts by weight per part by weight of the substrates.

Depending on the method of charging the substrates, the hydrophobic organic solvent (D) may be wholly charged from the beginning of the reaction, or can be charged continuously or intermittently along with the pre-reaction mixture which is charged continuously or intermittently.

The acid catalyst (E) may be wholly charged into the reactor from the beginning or may be charged continuously or intermittently through a separate line independently. Alternatively, the catalyst can be charged as added to the pre-reaction mixture. The amount of acid catalyst (E) to be used is not limited specifically but can be determined from a wide range. It is generally about 0.05 to about 10 parts by weight, preferably about 0.2 to about 3 parts by weight, per 100 parts by weight of the substrates.

The concentration of substrates (wt. %) expressed by the formula 100 x S/(S + H) wherein S is the weight of substrates, and H is the weight of hydrophobic organic solvent can be determined suitably from the wide range of about 5 to about 90 wt. %. The reaction system can be set in the state of a slurry of low viscosity, the state of paste or the state of powder by adjusting the amount of hydrophobic organic solvent to be used in accordance with the type of the reactor used to thereby select the concentration of substrates. If the substrate concentration is lower than about 5 wt. %, a slightly decelerated reaction tends to result, whereas concentrations in excess of about 90 wt. % tend to lower the selectivity.

In the case where the substrate concentration is up to about 20 wt. %, the reaction system is in the state of slurry and can therefore be stirred by usual stirring blades, so that the whole amount of substrates can be charged from the beginning of the reaction. When the substrate concentration is not lower than about 20 wt. %, the pre-reaction mixture can be charged intermittently or continuously to thereby maintain the system in a steady state of reaction without entailing a sudden increase in the load on the usual stirring blades.

The reaction temperature, which is variable, for example, with the kind of hydrophobic organic solvent, is maintained at a level which is effective for distilling off the lower alcohol (C) and water from the reaction system as an azeotropic mixture or by vapor-liquid equilibration. Generally, the reaction temperature is suitably determined from the range of about 40 to about 200°C, preferably about 40 to about 130°C. Preferably it is usually the reflux temperature.

The reaction is conducted preferably in nitrogen gas or like inert gas atmosphere to prevent the coloration of the desired product and to assure the safety of reaction.

In carrying out the condensation reaction of the present process to be conducted under such conditions, it is essential that the lower alcohol (C) be dividedly charged into the reactor at least three times, and that more than one-half of the amount of the lower alcohol and the water produced with the progress of condensation reaction be withdrawn from the reaction system each time by vapor-liquid equilibration or as an azeotropic mixture. The present invention involves several methods of dividedly charging the lower alcohol as will be described below.

In a preferred embodiment of the invention, the lower alcohol is dividedly charged into the reactor by the following method. The whole amount of lower alcohol (C) to be used in this embodiment can be suitably determined from the range of about 40 to about 500 parts by weight, preferably about 50 to about 400 parts by weight, per 100 parts by weight of the substrates (aldehyde compound (A) + polyhydric alcohol (B)). First, the aldehyde compound (A), polyhydric alcohol (B), hydrophobic organic solvent (D) and acid catalyst (E) are charged, each in the whole amount, into the reactor from the beginning of the reaction. The lower alcohol (C) is also charged in from the beginning of the reaction in an amount of about 20 to about 350 parts by weight, preferably about 30 to about 100 parts by weight, per 100 parts by weight of the substrates. The charge of lower alcohol (C) in the beginning of the reaction is defined as the first charge alcohol. After more than one-half of the initial charge of lower alcohol (C) has been drawn off along with condensation water with the progress of reaction, the lower alcohol (C) is charged the second time. Each of the second and subsequent charges is suitably determined from the range of about 5 to about 100 wt. %, preferably about 10 to about 30 wt. %, based on the initial charge of substrates. In this way, the lower alcohol (C) is dividedly added at least three times before the completion of the reaction, and more than one-half of the charge is drawn off each time along with water. Usually the lower alcohol is added three to about 15 times before the completion of the reaction.

In another preferred embodiment of the invention, the lower alcohol is dividedly charged during continuous or intermittent charging of the pre-reaction mixture or after the completion of the continuous or intermittent charging. First, the aldehyde compound (A), polyhydric alcohol (B), lower alcohol (C) and, when required, the acid catalyst (E) are mixed together in the foregoing proportions at room temperature to a temperature of about 80°C to prepare a pre-reaction mixture. Next, the pre-reaction mixture and the hydrophobic organic solvent are continuously or intermittently charged into the reactor and stirred for reaction as disclosed in European Patent Laid-Open Application No. 319917 (EP 0319917A2). The pre-reaction mixture is continuously or intermittently charged at such a rate that the substrates (i.e., aldehyde compound (A) and polyhydric alcohol (B)) will be fed in a combined amount per hour of about 0.1 to about 2 parts by weight, preferably about 0.3 to about 1 part by weight, per part by weight of the acetal compound of the formula (I) present in the reaction system. This charging of the pre-reaction mixture will be defined as the first charge of lower alcohol. After more than one-half of the initial charge of lower alcohol (C) has been drawn off along with condensation water with the progress of reaction, the lower alcohol is charged the second time and subsequently. The lower alcohol may be charged and withdrawn the second time and subsequently after the entire amount of pre-reaction mixture has been completely charged, or alternatively during the charging of the pre-reaction mixture upon interruption of the charging. For example, a given proportion (e.g. about 50 wt. %) of the pre-reaction mixture is charged, and more than one-half of the charge of lower alcohol is drawn off along with condensation water with the progress of reaction. Subsequently, the procedure for drawing off the lower alcohol and water after charging the lower alcohol according to the invention is repeated until the products in the reaction system are almost entirely converted to the desired 1,3;2,4-diacetal compound. The remainder of the pre-reaciton mixture is thereafter continuously or intermittently charged into the reactor along with the hydrophobic organic solvent at the same rate as previously or at a higher rate, followed by further reaction with stirring. When more than one-half of the amount of lower alcohol has been withdrawn with the progress of reaction, the procedure of the invention for drawing off the lower alcohol and water after charging the lower alcohol is repeated again until the products in the reaction system are almost entirely converted to the desired 1,3;2,4-diacetal compound. Each of the second and subsequent charges of lower alcohol (C) is suitably determined from the range of 5 to 100 wt. %, preferably 10 to 30 wt. %, based on the amount of substrates already charged by the time the alcohol is to be added. In this way, the lower alcohol (C) is added dividedly at least three times before the completion of the reaction. Usually, the lower alcohol is added three to about 15 times before the completion of the reaction.

Thus according to the invention, it is essential that the lower alcohol (C) dividedly added be withdrawn from the reaction system along with the condensation water produced with the progess of reaction. The lower alcohol and the water distill from the reaction system in the form of an azerotropic mixture of these substances and the hydrophobic organic solvent (D) or owing to the vapor-liquid equilibration of the three substances. When the distillate is condensed with a condenser having a decanter, the condensate separates into two layers. The upper layer consists primarily of the hydrophobic organic solvent (D), while the lower layer consists essentially of a mixture of the lower alcohol (C) and water. The upper layer and the lower layer are drawn off from the reactor. The organic solvent (D) contained in the upper layer may be recycled to the reaction system.

The lower alcohol (C) dividedly added to the reaction system is drawn off from the reaction system along with water with the progress of reaction as described above, so that the amount of lower alcohol (C) in the reaction system decreases. When more than one-half, more specifically about 60 to about 95 wt. %, of the single charge of lower alcohol is drawn off, the next charge of lower alcohol is added. When to add this subsequent charge can be easily determined by monitoring the temperature of the reaction system. More specifically stated, the temperature of the reaction system approaches the boiling point of the hydrophobic organic solvent (D) with a decrease in the amount of lower alcohol (C) in the system since the system primarily contains the hydrophobic organic solvent (D). At this time, the subsequent charge of lower alcohol is placed in.

The desired acetal compound of the formula (I) can be produced in a good yield with high selectivity by repeating at least three times the procedure of adding a divided portion of the lower alcohol and withdrawing the alcohol from the reaction system along with water in the manner described above to carry out the reaction. While the procedure of dividedly adding the lower alcohol and drawing off the alcohol from the reaction system along with water needs to be repeated at least three times, the reaction is terminated when the desired selectivity and yield are achieved. The reaction time is generally about 2 to about 10 hours, preferably about 3 to about 6 hours.

The reaction mixture thus obtained is in the form of a slurry or wet powder. When required, a neutralizing agent, such as an aqueous solution or water-containing lower alcohol solution of sodium hydroxide or potassium hydroxide, is added to the reaction mixture as it is or as filtered to neutralize the acid catalyst. The mixture is then washed with water at room temperature to about 90°C to remove the unreacted substrates, intermediate reaction products, etc., followed by drying and, when desired, by pulverization to obtain a product.

The present invention has the following outstanding advantages.
(1) The reaction achieves a high efficiency and can be completed within a short period of time.
(2) The desired acetal compound of the formula (I) is available with high selectivity.
(3) Greatly inhibited formation of thermally unstable dioxolan-type isomers as by-products.
(4) The reaction conducted by repeating the procedure of dividedly adding the lower alcohol and withdrawing the alcohol from the reaction system along with water gives the desired acetal compound of the formula (I) in an improved yield. When this procedure is repeated a sufficient number of times, the desired actal compound of the formula (I) is obtained in an almost quantitative yield.
(5) The aggregate formed as a by-product during the condensation reaction diffuses through the system to prevent formation of a gel.
(6) Since the dioxane-type isomer is exceedingly superior to other isomers in thermal stability, the product obtained has remarkably improved thermal stability.

The reason why the present invention achieves such outstanding effects still remains to be fully clarified, but these effects appear attributable to the following. In this type of condensation reaction, formation of the aforementioned mono- or di-dioxane-type isomers and formation of the mono-, di- or tri-dioxolan-type isomers take place as competitive reactions, which afford these two types of isomers in a certain ratio. Generally, the dioxolan-type isomers have a low melting point, are soluble in the hydrophobic organc solvent (D) and are therefore present in the liquid phase (homogeneous phase) of the reaction system. When the lower alcohol (C) is caused to be present in the homogeneous phase, the dioxolan-type isomers will presumably be converted to dioxane-type isomers at a high velocity. If the lower alcohol (C) is drawn off from the reaction system, the conversion reaction velocity decreases, whereas addition of the lower alcohol (C) to the system gives rise to the conversion reaction again at a high velocity. Accordingly, every time the addition and withdrawal of the lower alcohol (C) are repeated as in the present invention, dioxolan-type isomers are converted to dioxane-type isomers at a high rate. At the same time, an unreacted portion of the polyhydric alcohol (B) reacts with an unreacted portion of the aldehyde compound (A) in the presence of the lower alcohol to form dioxane-type isomers in addition to the dioxolan-type isomers. On the other hand, the dioxane-type isomers have a high melting point and are insoluble in the hydrophobic organic solvent (D), so that the dioxane-type isomers formed immediately precipitate out in the reaction system. Of the dioxane-type isomers precipitating out, the mono-dioxane-type isomer reacts with the aldehyde compound (A) in the presence of the lower alcohol (C) to change to the desired di-dioxane-type isomer.

In contrast, if the lower alcohol (C) is present in a large amount in the reaction system from the beginning of the reaction, the rate of distillation of the condensation water from the reaction system is very small. As a result, the conversion reaction of di-dioxolan-type isomer to di-dioxane-type isomer fails to proceed to a full extent owing to the influence of water present in the reaction system. Further because of the low water distillation efficiency, the reaction efficiency also tends to be lower. Presumably for this reason, the yield of the desired di-dioxane-type isomer remains below a fully satisfactory level even if the reaction is conducted for three times the reaction time of the invention, and yet the reaction forms considerable amounts of mono-dioxane-type isomer and dioxolan-type isomers (see Comparative Examples to follow).

The present invention will be described in greater detail with reference to the following examples and comparative examples.

### Example 1

(1) Into a two-liter four-necked reactor equipped with a thermometer, stirrer, nitrogen gas inlet and condenser having a decanter were placed 38 g (0.21 mole) of finely divided sorbitol, 44 g (0.42 mole) of benzaldehyde, 700 ml of cyclohexane, 5 g of 50% sulfuric acid and 70 ml of methanol, which were heated with stirring. With a rise in the temperature of the reaction system, the system started reflux. The methanol distilled off along with water produced as a by-product was condensed in the condenser having the decanter during the reaction and drawn off in the form of a methanol/water mixture. As the methanol/water mixture was drawn off, the temperature of the reaction system rose. Upon the temperature of the reaction system reaching 75°C, 15 ml of methanol was added to the system. The procedure of adding 15 ml of methanol was thereafter repeated similarly when the temperature of the reaction system reached 75°C.

When the reaction was continued for an overall period of 2.5 hours during which the procedure of adding 15 ml of methanol was repeated five times, a portion of the reaction mixture was withdrawn, dissolved in pyridine and then analyzed by gas chromatography. On the other hand, the remaining portion of the reaction mixture was neutralized with a water-containing methanol solution of sodium hydroxide, washed with water and dried to give a purified product, which was then analyzed by high-performance liquid chromatography (HPLC).
(2) An acetal product was obtained by the same process as process (1) above except that the reaction was continued for an overall period of 3.5 hours during which the procedure of adding 15 ml of methanol was repeated 10 times.
(3) An acetal product was obtained by the same process as process (1) above except that the reaction was continued for an overall period of 4.5 hours during which the procedure of adding 15 ml of methanol was repeated 12 times.

The compositions (wt. %) of the acetal products obtained by processes (2) and (3) were analyzed in the same manner as in process (1). Table 1 below shows the result of analyses along with the reaction conditions.

### Example 2

An acetal product was prepared in the same manner as in Example 1 with the exception of using 49.8 g (0.42 mole) of p-methylbenzaldehyde in place of benzaldehyde. More specifically, 79.9 g of 1,3;2,4-p,p'-dimethylbenzylidenesorbitol was obtained in a yield of 99.2% by using methanol in a total amount of 220 ml and repeating the procedure of adding 15 ml of methanol 10 times during the reaction time of 3.5 hours.

### Example 3

An acetal product was prepared in the same manner as in Example 1 with the exception of using 56.5 g (0.42 mole) of p-ethylbenzaldehyde in place of benzaldehyde. More specifically, 86.2 g of 1,3;2,4-p,p'-diethylbenzylidenesorbitol was obtained in a yield of 99.8% by using methanol in a total amount of 220 ml and repeating the procedure of adding 15 ml of methanol 10 times during the reaction time of 3 hours.

### Comparative Example 1

An acetal product was prepared in the same manner as in Example 1 with the exception of charging 250 ml of methanol into the reactor at a time when the reaction was to be started without adding any methanol during the reaction. The reaction was conducted (1) for 3 hours or (2) for 6 hours, giving 45 g of the acetal product with the composition shown in Table 1 (yield 60%).

Table 1 shows the reaction conditions and the composition of the acetal product as determined in the same manner as in Example 1.

### Comparative Example 2

An acetal product was prepared in the same manner as in Example 1 with the exception of charging 500 ml of methanol into the reactor at a time when the reaction was to be started without adding any methanol during the reaction. The reaction was conducted for 8 hours, giving 49 g of the acetal product with the composition shown in Table 1 (yield 65%).

Table 1 shows the reaction conditions and the composition of the acetal product as determined in the same manner as in Example 1.

### Comparative Example 3

An acetal product was prepared in the same manner as in Example 1 with the exception of charging 800 ml of methanol into the reactor at a time when the reaction was to be started without adding any methanol during the reaction. The reaction was conducted for 10 hours, giving 64 g of the acetal product with the composition shown in Table 1 (yield 85%).

Table 1 shows the reaction conditions and the composition of the acetal product as determined in the same manner as in Example 1.

### Comparative Example 4

An acetal product was prepared in the same manner as in Example 1 with the exception of charging 900 ml of methanol into the reactor at a time when the reaction was to be started without adding any methanol during the reaction. The reaction was conducted for 12 hours, giving 68 g of the acetal product with the composition shown in Table 1 (yield 91%).

Table 1 shows the reaction conditions and the composition of the acetal product as determined in the same manner as in Example 1.

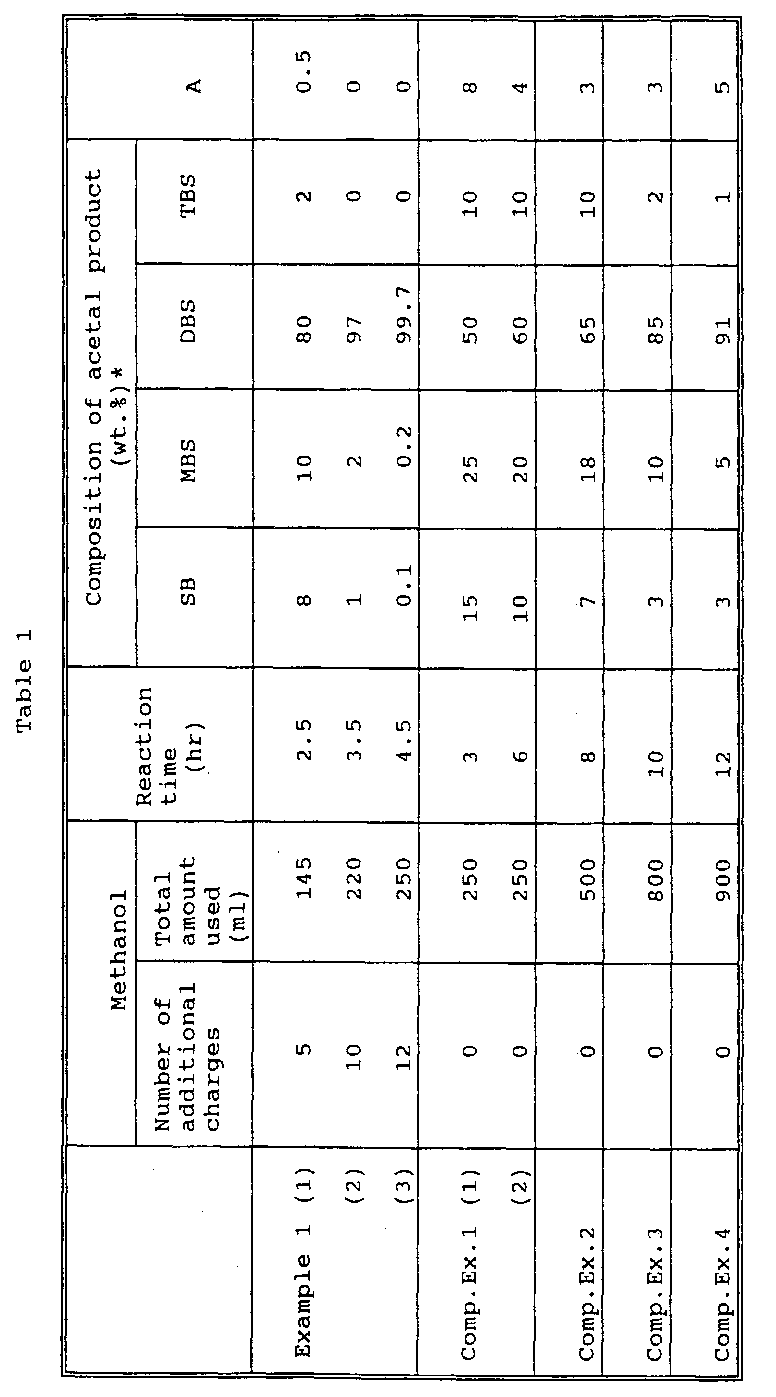

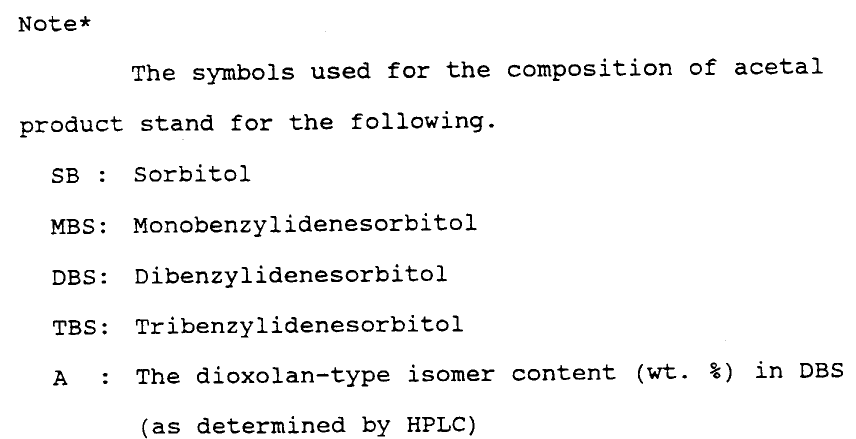

Table 1 reveals that the present invention affords the desired acetal compound (1,3;2,4-didioxane-type isomer) within a short period of time and with a high efficiency and high purity.

## Claims

1. A process for producing an acetal compound represented by the general formula 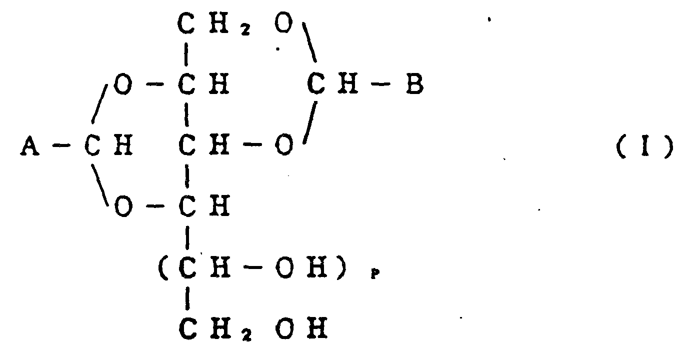 wherein A and B are the same or different and are each phenyl, naphthyl or tetrahydronaphthyl, the phenyl, naphthyl or tetrahydronaphthyl having or not having 1 to 5 substitutents selected from among C₁-C₄ alkyl group, C₁-C₄ alkoxy group, halogen atom, carboxyl group, (C₁-C₂₀ alkylozy)carbonyl group, (C₁-C₂₀ alkyloxy)ethylozycarbonyl group, (C₁-C₁₂ alkyl)phenyl group, halogenated phenyl group, (C₁-C₁₂ alkoxy)phenyl group, (C₁-C₁₂ alkyloxy)ethyloxyethylozycarbonyl group and (C₁-C₁₂ alkylozy)ethyloxyethylozyethyloxycarbonyl group, and p is 0 or 1 by subjecting (%) at least one of aldehyde compounds represented by the general formula
A-CHO (II)
and the general formula
B-CHO (III)
wherein A and B are as defined above, and (B) a polyhydric alcohol selected from among a pentahydric alcohol and a hexahydric alcohol to condensation in the presence of (C) a lower alcohol selected from the group consisting of a saturated aliphatic alcohol having 1 to 4 carbon atoms, allyl alcohol or furfuryl alcohol, (D) a hydrophobic organic solvent not forming a gel in any way or not forming a tough gel with the acetal compound of the general formula (I) prepared, serviceable as a dispersion medium and having a boiling point of about 40 to about 200°C and (E) an acid catalyst, the process being characterized in that the procedure of charging the lower alcohol into a reactor and thereafter withdrawing more than one-half of the charge of lower alcohol from the reaction system along with water is repeated at least three times during the condensation reaction.

2. A production process as defined in claim 1 wherein the aldehyde compounds (A) of the general formulae (II) and (III) are benzaldehyde having or not having p-methyl, m-methyl, o-methyl, p-ethyl, p-isopropyl, p-butyl, p-isobutyl, p-phenyl, p-(C₁-C₁₂ alkyl)phenyl, p-(halogenated phenyl), p-(C₁-C₁₂ alkoxy)phenyl, 2,4-dimethyl, 2,4,5-trimethyl, p-chloro, p-methoxy, p-fluoro, p-methoxy-carbonyl, p-hexyloxycarbonyl, p-butyloxyethyloxycarbonyl or p-methyloxyethyloxyethyloxycarbonyl, and unsubstituted tetrahydronaphthylaldehyde.

3. A production process as defined in claim 1 wherein the polyhydric alcohol (B) is an alcohol represented by the general formula 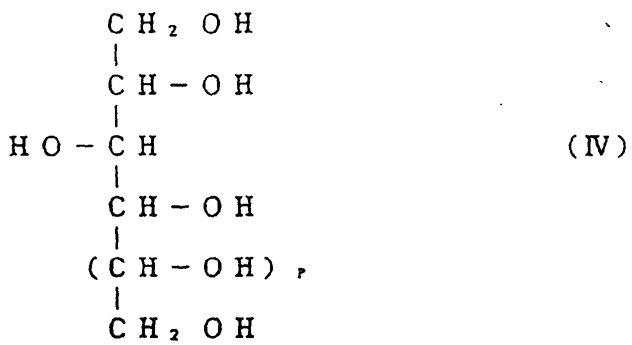 wherein p is 0 or 1.

4. A production process as defined in claim 1 wherein the polyhydric alcohol (B) is sorbitol or xylitol.

5. A production process as defined in claim 1 wherein the hydrophobic organic solvent (D) is benzene which may be substituted with one to three C₁-C₄ alkyl groups, cyclohexane which may be substituted with one to three C₁-C₄ alkyl groups or a straight- or branched-chain saturated hydrocarbon having 6 to 16 carbon atoms.

6. A production process as defined in claim 1 wherein the lower alcohol (C) is methanol.

7. A production process as defined in claim 1 wherein the acid catalyst (E) is sulfuric acid, phosphoric acid, hydrochloric acid, p-toluenesulfonic acid, m-toluenesulfonic acid, a benzenesulfonic acid substituted with an alkyl group having 2 to 18 carbon atoms, naphthalenesulfonic acid, G acid, R acid or zinc chloride.

8. A production process as defined in claim 1 wherein the aldehyde compound (A) and the polyhydric alcohol (B) are charged into the reactor in a mole ratio (A):(B) = about 1-4:1.

9. A production process as defined in claim 1 wherein the aldehyde compound (A) and the polyhydric alcohol (B) are charged into the reactor in a mole ratio (A):(B) = about 1.5-3:1.

10. A production process as defined in claim 1 wherein the aldehyde compound (A) and the polyhydric alcohol (B) are charged into the reactor in a mole ratio (A):(B) = about 1.8-2.2:1.

11. A production process as defined in claim 1 wherein the condensation reaction is conducted at a substrate concentration of about 5 to about 90 wt. %.

12. A production process as defined in claim 1 wherein the condensation reaction is conducted at a temperature of about 40 to about 200°C.

13. A production process as defined in claim 1 wherein the condensation reaction is conducted in an inert gas atmosphere.

14. A production process as defined in claim 1 wherein the aldehyde compound, the polyhydric alcohol, the hydrophobic organic solvent, the acid catalyst and about 20 to about 350 parts by weight of the lower alcohol per 100 parts by weight of the substrates are charged into the reactor from the beginning of the reaction, and more than one-half of the amount of the lower alcohol is distilled off along with water, each of the second and subsequent charges of the lower alcohol to be thereafter added being about 5 to about 100 wt. % based on the initial charge of the substrates.

15. A production process as defined in claim 14 wherein each of the second and subsequent charges of the lower alcohol is about 10 to about 30 wt. % based on the initial charge of the substrates.

16. A production process as defined in claim 1 wherein a uniform solution or suspension obtained by mixing together the aldehyde compound, the polyhydric alcohol, the lower alcohol and, when required, the acid catalyst at a temperature of room temperature to about 80°C is continuously or intermittently charged into the reactor along with the hydrophobic organic solvent, and more than one-half of the amount of lower alcohol is drawn off, each of the second and subsequent charges of the lower alcohol to be thereafter added being in the range of about 5 to about 100 wt. % based on the amount of substrates which has been charged.

17. A production process as defined in claims 1 and 16 wherein each of the second and subsequent charges of the lower alcohol is in the range of about 10 to about 30 wt. % based on the amount of substrates which has been charged.

18. A production process as defined in claim 17 wherein the lower alcohol is charged into the reactor upon interruption of the charging of a pre-reaction mixture after more than one-half of the amount of lower alcohol is drawn off, or after more than one-half of the amount of lower alcohol is drawn off following the completion of charging of the pre-reaction mixture.

19. A production process as defined in claim 1 wherein when about 60 to about 95 wt. % of the lower alcohol charged has been drawn off from the reaction system, the next charge of lower alcohol is added to the system.

20. A production process as defined in claim 19 wherein the next charge of lower alcohol is added when the temperature of the reaction system approaches the boiling temperature of the hydrophobic organic solvent in the reaction system.

## Patentansprüche

1. Verfahren zur Herstellung einer Acetalverbindung der allgemeinen Formel 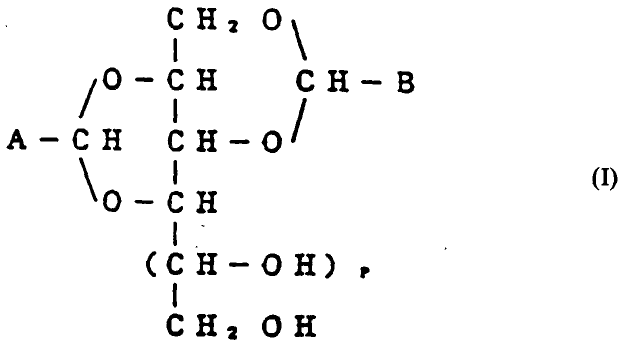 wobei A und B gleich oder verschieden sind und jeweils eine Phenyl-, Naphthyl- oder Tetrahydronaphthylgruppen darstellen, wobei die Phenyl-, Naphthyl- oder Tetrahydronaphthylgruppe entweder 1 bis 5 Substituenten besitzt oder nicht, gewählt unter C₁-C₄-Alkyl-, C₁-C₄-Alkoxyresten, Halogenatomen, Carboxyl-, (C₁-C₂₀-ALkyLoxy)carbonyl-, (C₁-C₂₀-Alkyloxy)ethyloxycarbonyl-, (C₁-C₁₂-Alkyl)-phenyl-, halogenierten Phenyl-, (C₁-C₁₂-Alkoxy)phenyl-, (C₁-C₁₂-Alkyloxy)ethyloxyethyloxycarbonyl- und (C₁-C₁₂-Alkyloxy)ethyloxyethyloxyethyloxycarbonylresten, und p für 0 oder 1 steht, indem
(A) wenigstens eine Aldehydverbindung der allgemeinen Formel
A-CHO (II)
und der allgemeinen Formel
B-CHO (III)
wobei A und B wie vorstehend definiert sind,
und
(B) ein mehrwertiger Alkohol, gewählt aus einem fünfwertigen Alkohol und einem sechswertigen Alkohol,
in Gegenwart
(C) eines Niederalkohols, gewählt aus der Gruppe, bestehend aus einem gesättigten, aliphatischen Alkohol mit 1 bis 4 Kohlenstoffatomen, Allylalkohol oder Furfurylalkohol,
(D) eines hydrophoben organischen Lösungsmittels, das in keiner Weise ein Gel erzeugt oder mit der hergestellten Acetalverbindung der allgemeinen Formel (I) kein beständiges Gel erzeugt, wobei das Lösungsmittel als ein Dispersionsmedium verwendbar ist und einen Siedepunkt von etwa 40 bis etwa 200°C besitzt,
und
(E) eines sauren Katalysators
kondensiert wird,
wobei das Verfahren dadurch gekennzeichnet ist, daß der Arbeitsgang des Beladens mit Niederalkohol in den Reaktor und danach das Abziehen von mehr als einer halben Ladung des Niederalkohols aus dem Reaktionssystem gemeinsam mit Wasser während der Kondensation wenigstens dreimal wiederholt wird.

2. Herstellungsverfahren nach Anspruch 1, wobei die Aldehydverbindungen (A) der allgemeinen Formeln (II) und (III) Benzaldehyd mit oder ohne p-Methyl-, m-Methyl-, o-Methyl-, p-Ethyl-, p-Isopropyl-, p-Butyl-, p-Isobutyl-, p-Phenylgruppen, p-(C₁-C₁₂-Alkyl)phenyl-, p-halogeniertem Phenyl-, p-(C₁-C₁₂-Alkoxy)phenylresten, 2,4-Dimethyl-, 2,4,5-Trimethyl-, p-Chlor-, p-Methoxy-, p-Fluor-, p-Methoxycarbonyl-, p-Hexyloxycarbonyl-, p-Butyloxyethyloxycarbonyl- oder p-Methyloxyethyloxyethyloxycarbonyl-gruppen sowie unsubstituieren Tetrahydronaphthylaldehyd darstellen.

3. Herstellungsverfahren nach Anspruch 1, wobei der mehrwertige Alkohol (B) einen Alkohol der allgemeinen Formel 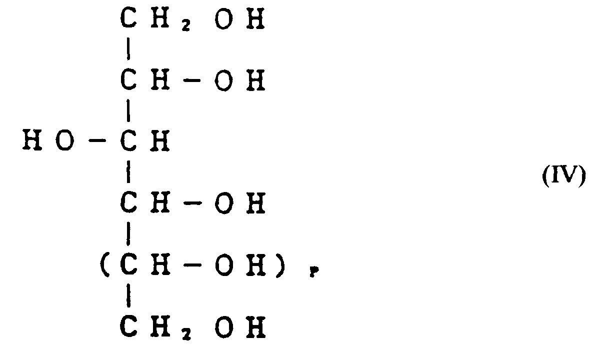 wobei p für 0 oder 1 steht, darstellt.

4. Herstellungsverfahren nach Anspruch 1, wobei der mehrwertige Alkohol (B) Sorbit oder Xylit ist.

5. Herstellungsverfahren nach Anspruch 1, wobei das hydrophobe organische Lösungsmittel (D) Benzol das mit ein bis drei C₁-C₄-Alkylresten substituiert sein kann, Cyclohexan, das mit ein bis drei C₁-C₄-Alkylresten substituiert sein kann, oder einen gerad- oder verzweigtkettigen, gesättigten Kohlenwasserstoff mit 6 bis 16 Kohlenstoffatomen darstellt.

6. Herstellungsverfahren nach Anspruch 1, wobei der Niederalkohol (C) Methanol ist.

7. Herstellungsverfahren nach Anspruch 1, wobei der saure Katalysator (E) Schwefelsäure, Phosphorsäure, Salzsäure, p-Toluolsulfonsäure, m-Toluolsulfonsäure, eine Benzolsulfonsäure, die mit einem Alkylrest mit 2 bis 18 Kohlenstoffatomen substituiert ist, Naphthalinsulfonsäure, G-Säure, R-Säure oder Zinkchlorid ist.

8. Herstellungsverfahren nach Anspruch 1, wobei die Aldehydverbindung (A) und der mehrwertige Alkohol (B) in den Reaktor in einem Molverhältnis von (A):(B) = etwa 1 bis 4:1 eingefüllt werden.

9. Herstellungsverfahren nach Anspruch 1, wobei die Aldehydverbindung (A) und der mehrwertige Alkohol (B) in den Reaktor in einem Molverhältnis von (A):(B) = etwa 1,5 bis 3:1 eingefüllt werden.

10. Herstellungsverfahren nach Anspruch 1, wobei die Aldehydverbindung (A) und der mehrwertige Alkohol (B) in den Reaktor in einem Molverhältnis von (A):(B) = etwa 1,8 bis 2,2:1 eingefüllt werden.

11. Herstellungsverfahren nach Anspruch 1, wobei die Kondensation bei einer Substratkonzentration von etwa 5 bis etwa 90 Gew.-% durchgeführt wird.

12. Herstellungsverfahren nach Anspruch 1, wobei die Kondensation bei einer Temperatur von etwa 40 bis etwa 200°C durchgeführt wird.

13. Herstellungsverfahren nach Anspruch 1, wobei die Kondensation in einer Inertgasatmosphäre durchgeführt wird.

14. Herstellungsverfahren nach Anspruch 1, wobei die Aldehydverbindung, der mehrwertige Alkohol, das hydrophobe organische Lösungsmittel der saure Katalysator und etwa 20 bis etwa 350 Gewichtsteile des Niederalkohols je 100 Gewichtsteile der Substrate zu Beginn der Umsetzung in den Reaktor eingefüllt werden, und mehr als die Hälfte der Menge an Niederalkohol gemeinsam mit Wasser abdestilliert wird, wobei jedes zweite und nachfolgende Beladen mit dem danach zuzugebenden Niederalkohol etwa 5 bis etwa 100 Gew.-%, bezogen auf die Anfangsbeladung der Substrate, beträgt.

15. Herstellungsverfahren nach Anspruch 14, wobei jedes zweite und nachfolgende Beladen mit Niederalkohol etwa 10 bis etwa 30 Gew.-%, bezogen auf die Anfangsbeladung der Substrate, beträgt.

16. Herstellungsverfahren nach Anspruch 1, wobei eine einheitliche Lösung oder Suspension, die durch Zusammenmischen der Aldehydverbindung, des mehrwertigen Alkohols, des Niederalkohols und, falls erforderlich, des sauren Katalysators bei Zimmertemperatur bis etwa 80°C erhalten wird, kontinuierlich oder diskontinuierlich gemeinsam mit dem hydrophoben organischen Lösungsmittel in den Reaktor eingefüllt wird, und mehr als die Hälfte der Menge an Niederalkohol abgezogen wird, wobei jedes zweite und nachfolgende Beladen mit dem danach zuzugebenden Niederalkohol im Bereich von etwa 5 bis etwa 100 Gew.-%, bezogen auf die Menge der eingefüllten Substrate liegt .

17. Herstellungsverfahren nach Anspruch 1 und 16, wobei jedes zweite und nachfolgende Beladen mit Niederalkohol im Bereich von etwa 10 bis etwa 30 Gew.-%, bezogen auf die Menge der eingefüllten Substrate liegt.

18. Herstellungsverfahren nach Anspruch 17, wobei der Niederalkohol in den Reaktor beim Unterbrechen des Beladens mit einem Vorreaktionsgemisch eingefüllt wird, nachdem mehr als die Hälfte der Menge an Niederalkohol abgezogen wurde oder nachdem mehr als die Hälfte der Menge an Niederalkohol abgezogen wurde, gefolgt vom Beenden des Beladens mit dem Vorreaktionsgemisch.

19. Herstellungsverfahren nach Anspruch 1, wobei die nächste Ladung Niederalkohol dem System zugegeben wird, wenn etwa 60 bis etwa 95 Gew.-% des eingefüllten Niederalkohols aus dem Reaktionssystem abgezogen worden sind.

20. Herstellungsverfahren nach Anspruch 19, wobei die nächste Ladung Niederalkohol zugegeben wird, wenn sich die Temperatur im Reaktionssystem der Siedetemperatur des hydrophoben organischen Lösungsmittels im Reaktionssystem nähert.

## Revendications

1. Procédé pour la production d'un composé acétal représenté par la formule générale : 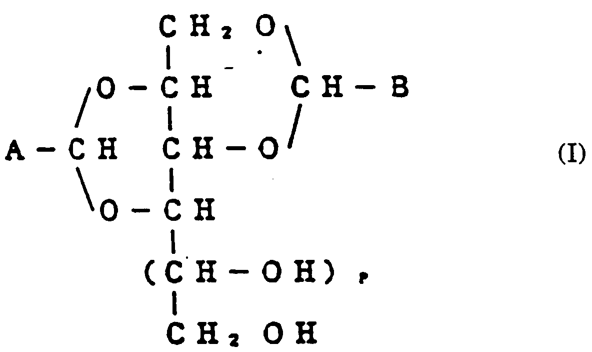 dans laquelle A et B sont identiques ou différents et représentent chacun un groupe phényle, un groupe naphtyle ou un groupe tétrahydronaphtyle, le groupe phényle, le groupe naphtyle ou le groupe tétrahydronaphtyle portant ou ne portant pas 1 à 5 substituants choisis parmi un groupe alkyle en C₁₋₄, un groupe alcoxy en C₁₋₄, un atome d'halogène, un groupe carboxy, un groupe alkyloxy-(en C₁₋₂₀)-carbonyle, un groupe alkyloxy-(en C₁₋₂₀)-éthyloxycarbonyle, un groupe alkyl-(en C₁₋₁₂)-phényle, un groupe phényle halogéné, un groupe alcoxy-(en C₁₋₁₂)-phényle, un groupe alkyloxy-(en C₁₋₁₂)-éthyloxyéthyloxycarbonyle, un groupe alkyloxy-(en C₁₋₁₂)-éthyloxyéthyloxyéthyloxycarbonyle, et p est 0 ou 1,
par soumission (A) d'au moins l'un des composés aldéhyde représentés par la formule générale :
A-CHO (II)
et la formule générale :
B-CHO (III)
dans lesquels A et B sont tels que définis plus haut, et (B) d'un alcool polyhydrique choisi parmi un alcool pentahydrique et un alcool hexahydrique, à une condensation en présence de (C) un alcool inférieur choisi dans le groupe consistant en un alcool aliphatique saturé ayant 1 à 4 atomes de carbone, l'alcool allylique ou l'alcool furfurylique, (D) un solvant organique hydrophobe ne formant aucunement un gel ou ne formant pas un gel résistant avec le composé acétal de formule générale (I) préparé, utilisable en tant que milieu de dispersion et ayant un point d'ébullition compris entre environ 40°C et environ 200°C et (E) un catalyseur acide,
le procédé étant caractérisé en ce que la procédure de charge de l'alcool inférieur dans un réacteur et ensuite de soutirage de plus d'une moitié de la charge d'alcool inférieur hors du système réactionnel avec de l'eau, est répétée au moins à trois reprises pendant la réaction de condensation.

2. Procédé de production suivant la revendication 1, dans lequel les composés aldéhyde (A) représentés par les formules générales (II) et (III) sont un benzaldéhyde portant ou ne portant pas des substituants p-méthyle, m-méthyle, o-méthyle, p-éthyle, p-isopropyle, p-butyle, p-isobutyle, p-phényle, p-alkyl-(en C₁₋₁₂)-phényle, p-phényle halogéné, p-alcoxy-(en C₁₋₁₂)-phényle, 2,4-dhnéthyle, 2,4,5-triméthyle, p-chloro, p-méthoxy, p-fluoro, p-méthoxy-carbonyle, p-hexyloxycarbonyle, p-butyloxyéthyloxycarbonyle ou p-méthyloxyéthyloxyéthyloxycarbonyle, et le tétrahydronaphtylaldéhyde non substitué.

3. Procédé de production suivant la revendication 1, dans lequel l'alcool polyhydrique (B) est un alcool représenté par la formule générale : 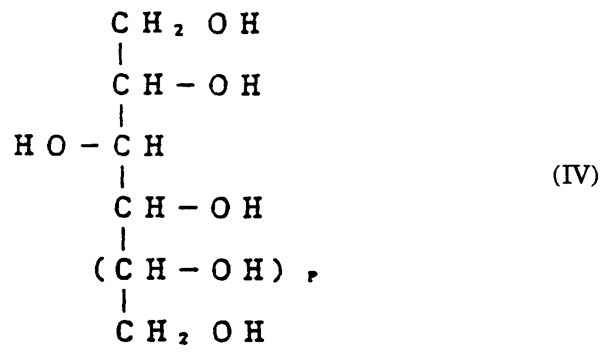 dans laquelle p est 0 ou 1.

4. Procédé de production suivant la revendication 1, dans lequel l'alcool polyhydrique (B) est le sorbitol ou le xylitol.

5. Procédé de production suivant la revendication 1, dans lequel le solvant organique hydrophobe (D) est le benzène qui peut être substitué avec un à trois groupes alkyles en C₁₋₄, le cyclohexane qui peut être substitué avec un à trois groupes alkyles en C₁₋₄, ou bien un hydrocarbure saturé à chaîne droite ou ranifiée ayant 6 à 16 atomes de carbone.

6. Procédé de production suivant la revendication 1, dans lequel l'alcool inférieur (C) est le méthanol.

7. Procédé de production suivant la revendication 1, dans lequel le catalyseur acide (E) est l'acide sulfurique, l'acide phosphorique, l'acide chlorhydrique, l'acide p-toluènesulfonique, l'acide m-toluènesulfonique, un acide benzènesulfonique substitué avec un groupe alkyle ayant 2 à 18 atomes de carbone, l'acide naphtalènesulfonique, l'acide G, l'acide R ou le chlorure de zinc.

8. Procédé de production suivant la revendication 1, dans lequel le composé aldéhyde (A) et l'alcool polyhydrique (B) sont chargés dans le réacteur selon un rapport molaire (A) : (B) d'environ 1 à 4 : 1.

9. Procédé de production suivant la revendication 1, dans lequel le composé aldéhyde (A) et l'alcool polyhydrique (B) sont chargés dans le réacteur selon un rapport molaire (A) : (B) d'environ 1,5 à 3 : 1.

10. Procédé de production suivant la revendication 1, dans lequel le composé aldéhyde (A) et l'alcool polyhydrique (B) sont chargés dans le réacteur selon un rapport molaire (A) : (B) d'environ 1,8 à 2,2 : 1.

11. Procédé de production suivant la revendication 1, dans lequel la réaction de condensation est conduite avec une concentration de substrats d'environ 5 à environ 90% en poids.

12. Procédé de production suivant la revendication 1, dans lequel la réaction de condensation est conduite à une température comprise entre environ 40°C et environ 200°C.

13. Procédé de production suivant la revendication 1, dans lequel la réaction de condensation est conduite dans une atmosphère de gaz inerte.

14. Procédé de production suivant la revendication 1, dans lequel le composé aldéhyde, l'alcool polyhydrique, le solvant organique hydrophobe, le catalyseur acide et environ 20 à environ 350 parties en poids de l'alcool inférieur pour 100 parties des substrats sont chargés dans le réacteur à partir du début de la réaction, et plus d'une moitié de la quantité de l'alcool inférieur est éliminée par distillation avec de l'eau, chacune de la deuxième charge et des charges ultérieures de l'alcool inférieur à ajouter par la suite représentant d'environ 5 à environ 100% en poids par rapport à la charge initiale des substrats.

15. Procédé de production suivant la revendication 14, dans lequel chacune de la deuxième charge et des charges ultérieures de l'alcool inférieur représente environ 10 à environ 30% en poids par rapport à la charge initiale des substrats.

16. Procédé de production suivant la revendication 1, dans lequel une solution ou une suspension uniforme obtenue par mélange du composé aldéhyde, de l'alcool polyhydrique, de l'alcool inférieur et, lorsque cela est nécessaire, du catalyseur acide, à une température comprise entre la température ambiante et environ 80°C, est chargée en continu ou par intermittence dans le réacteur en même temps que le solvant organique hydrophobe et plus d'une moitié de la quantité d'alcool inférieur est soutirée, chacune de la deuxième charge et des charges ultérieures de l'alcool inférieur à ajouter par la suite représentant d'environ 5 à environ 100% en poids par rapport à la quantité de substrats qui a été ajoutée.

17. Procédé de production suivant les revendications 1 et 16, dans lequel chacune de la deuxième charge et des charges ultérieures de l'alcool inférieur est dans la gamme d'environ 10 à environ 30% en poids par rapport à la quantité de substrats qui a été chargée.

18. Procédé de production suivant la revendication 17, dans lequel l'alcool inférieur est chargé dans le réacteur lors d'une interruption de l'introduction d'un mélange pré-réactionnel après soutirage de plus d'une moitié de la quantité d'alcool inférieur, ou après soutirage de plus d'une moitié de la quantité d'alcool inférieur à la suite de l'achèvement de l'introduction du mélange pré-réactionnel.

19. Procédé de production suivant la revendication 1, dans lequel la charge suivante d'alcool inférieur est ajoutée dans le système lorsqu'environ 60 à environ 95% en poids de l'alcool inférieur chargé ont été soutirés du système réactionnel.

20. Procédé de production suivant la revendication 19, dans lequel la charge suivante d'alcool inférieur est ajoutée lorsque la température du système réactionnel est voisine de la température d'ébullition du solvant organique hydrophobe dans le système réactionnel.
